# EUROPEAN PATENT SPECIFICATION

(11) **EP 3 506 874 B1**
(45) Date of publication and mention of the grant of the patent: **21.10.2020**
(21) Application number: 17754656.1
(22) Date of filing: 07.08.2017
(51) Int. Cl.: A61K 8/34, A61K 8/67, A61Q 11/00, A61Q 19/00, A61P 17/00

(54) **A PERSONAL CARE COMPOSITION**
KÖRPERPFLEGEZUSAMMENSETZUNG
COMPOSITION DE SOINS PERSONNELS

(30) Priority: 30.08.2016 WO PCT/CN2016/097313; 03.10.2016 EP 16192066
(43) Date of publication of application: 10.07.2019
(73) Proprietor: Unilever N.V., 3013 AL Rotterdam (NL); Unilever PLC, London, Greater London EC4Y 0DY (GB)
(72) Inventor: CHEN, Yu, Shanghai 200335 (CN); HARDING, Clive, Roderick, Little Neston Cheshire CH64 4AF (GB); HARICHIAN, Bijan, Trumbull Connecticut 06611 (US); HUANG, Dandan, Shanghai 200335 (CN); ROSA, Jose, Guillermo, Trumbull Connecticut 06611 (US); ZHOU, Luxian, Shanghai Minhang District 201114 (CN)
(74) Representative: Tansley, Sally Elizabeth
(86) International application number: PCT/EP2017/069920
(87) International publication number: WO 2018/041544

(56) References cited:
- DE-U1- 29 719 974
- US-A1- 2002 051 800
- US-A1- 2009 238 905
- US-A1- 2010 028 469
- FICKER C ET AL: "Bioassay-guided isolation and identification of antifungal compounds from ginger", MEDICINAL & AROMATIC PLANTS ABSTRACTS, SCIENTIFIC PUBLISHERS, NEW DELHI - INDIA, vol. 26, no. 1, 1 February 2004 (2004-02-01), XP018000358,

## Description

### Field of the Invention

The invention relates to a personal care composition, more particularly to a composition for application to a topical surface or oral cavity of a human or animal that prevents or reduces inflammation. The composition could be delivered in the form of a skin, scalp hair or oral care product.

### Background of the Invention

Inflammation, a complicated biological host response to harmful stimuli, is a mechanism by which the host removes the stimuli and initiates the healing process for self-protection. The innate immune system for a host is the first line of defence against invading organisms in a non-specific manner. Dysregulated inflammation may cause various personal care problems including gingivitis/periodontitis (in the oral cavity), dandruff (on scalp/ hair) and eczema/acnes (on skin). To assist the host organism (e.g. the human or animal) several anti-inflammatory agents either through topical application or through oral consumption have been developed and used to mitigate the above problems.

Many products that we consume have a negative impact on our teeth and mouth. Acidic drinks and sweets, for example, can result in tooth erosion by attacking enamel which is the outer coating that protects the teeth. Foods which are sticky can accumulate in the interstices between teeth and in the region between the gums and the teeth. In the absence of good oral hygiene, these can harden over time with the help of oral bacteria to form a film known as plaque.

Gingivitis is an inflammatory process of the gums caused by accumulation of plaque and/or bacteria. During gingivitis, the bacteria residing in the dental plaque biofilms and its corresponding components interact with gingival tissues. Following this, innate immune response is activated, characterised by the release of pro-inflammatory cytokines. Gingivitis is a mild phase of periodontal disease and defined as reversible inflammation. It is believed that good habit of oral hygiene e.g. brushing and using mouthrinse product with therapeutic anti-microbial and anti-inflammatory efficacy can be an effective way for individuals to reduce the plaque build-up that causes gingivitis.

Chronic gingivitis results in mild bleeding from the gums during tooth brushing. Gingivitis can progress to a more severe state (chronic periodontitis) when the inflammatory process extends to the periodontal ligament and alveolar bone and/or exert a significant systemic impact on health. Chronic periodontitis is asymptomatic until teeth shift, loosen, or are lost.

Dandruff is a condition experienced by many people worldwide. The dandruff condition varies from mild symptoms such as flaking skin to severe inflammation and itchiness of the scalp. Malassezia yeasts, such as *Malassezia furfur,* are believed by some to be the main cause of dandruff and, whilst this might not represent the full scientific picture of the situation, Malassezia yeasts do appear to be closely associated with dandruff. Hence, the strategy conventionally used for the treatment of dandruff is the topical application of antifungals such as zinc pyrithione (ZnPTO), octopirox, climbazole and ketoconazole which are normally delivered through a shampoo. Additionally, anti-inflammatory agents have also been used in anti-dandruff products to alleviate the ill-effects of this condition.

On the skin, one of the problems experienced by many people, especially on the face, is acne. This has a displeasing cosmetic appearance. Acne, also known as Acne vulgaris, is a common skin condition that affects nearly all adolescents and adults at some times in their lives. It has a complex etiology, involving abnormal keratinization, excess sebum production, androgen function, bacterial growth, and immune hypersensitivity. Although one or more of the above processes is correlated with acne, the one triggering factor and the exact sequence of events leading to the formation of acne lesions has not been fully understood. Other factors which have been linked to acne are presence of free radicals with subsequent oxidative stress leading to cellular damage. It has been observed that acne usually occurs in areas rich in sebaceous glands like the face, neck and back. A bacteria *Propionibacterium acnes* (*P. acnes*) has also been implicated in occurrence of acne.

Acne has been treated in many ways. Most treatments take several weeks to months before a noticeable change is seen. Benzoyl peroxide which has an antibacterial effect has been used for mild cases of acne and is also believed to prevent formation of further acne. In very severe cases of acne, antibiotics like tetracycline, erythromycin and clindamycin have been used. Antibiotics are believed to work by several mechanisms, the most important being the decrease in the number of bacteria in and around the follicle. They are also thought to reduce the irritating chemicals produced by the white blood cells in the sebum, thereby reducing the inflammatory response.

Thus, inflammation is a process that is manifest on the topical surface of the human or animal body in one or all of the above described conditions. The present inventors have attempted to alleviate the symptoms of the above conditions by developing combination of actives that exhibit synergistic anti-inflammatory benefits. The present invention concerns identification of actives which could be derivatized from compounds found in extracts of natural materials. Natural materials from which many actives have been extracted include ginger, turmeric, tea, grape, tomato and a host of others. The present inventors have studied a compound gingerdiol (especially 6-gingerdiol) which is a derivative of a compound that is extracted from ginger viz. gingerol for its efficacy in reducing oral inflammation. The inventors have further studied the possible synergistic anti-inflammatory efficacy when this active is combined with various other cosmetically acceptable compounds like vitamins and minerals (e.g. vitamin B3).

KR100835899 (Korean Res. Inst of Bioscience, 2008) discloses an extract of *Zingiber officinale Roscoe* or gingerol isolated therefrom is provided to show antibacterial capability and sterilizing capability on *Porphyromonas endodontalis, Porphyromonas gingivalis and Prevotella intermedia,* thereby being usefully used for preventing or treating periodontal diseases. A pharmaceutical composition for preventing or treating periodontal diseases or a composition for cleaning oral cavity is provided which comprises a hexane extracted fraction of *Zingiber officinale Roscoe* or gingerol isolated from the *Zingiber officinale Roscoe,* which is 10-gingerol, or 12-gingerol. Further, the composition for cleaning oral cavity is used in a form of tooth paste, gargle solution, ointment, spray and dressing solution.

WO07/042472 (symrise) describes a formulation having a skin irritation-reducing action consisting of or comprising: bisabolol and a composition or compound chosen from the group consisting of a) substance mixtures obtainable from an extraction of ginger, b) substance mixtures obtainable from a separation of a ginger extract which comprise a compound which is chosen from the group consisting of gingerols, shogaols, gingerdiols, dehydrogingerdiones, paradols and derivatives thereof and c) compounds obtainable from a separation of a ginger extract which are chosen from the group consisting of gingerols, shogaols, gingerdiols, dehydrogingerdiones, paradols and derivatives thereof and mixtures thereof, wherein the particular content of bisabolol and of the said composition or compound in the formulation is adjusted such that the skin irritation-reducing action of these contents is increased synergistically.

DE 29719974 U1 (Doehler Euro Citrus Natural BE, 1998) discloses beverage composition comprising a base for the drink, 50 to 5,000 mg/l ginger extract, 27 to 270 mg/l niacin, 3 to 30 mg/l vitamin B6 and 40 to 400 mg/l caffeine.

US 2010/028469 A1 (Alberte et al.) discloses extracts of cranberry (Vaccinium macrocarpon) comprising an enriched amount of certain compounds having anti-infective activity, e.g. antibacterial and/or antifungal activity, e.g. activity against C. albicans.

US 2002/051800 A1 (Weidner) discloses a cosmetic composition for the suppression of inflammatory reaction having a mixture of specific fatty acids and the plant Zingiber officinale Roscoe or parts thereof or an extract or a component thereof.

US 2009/0238905 A1 (Gurney et al.) discloses a formulation for treating or eliminating irritation and/or inflammation-reducing effect on endodermal tissue of the respiratory tract, the gastrointestinal tract, or both which include bisabolol or an extract containing bisabolol and actives found in ginger or extracted from ginger.

The above publications do not disclose a combination of gingerdiol and vitamin B3 as an active for reducing inflammation and therefore as an active which may be used in oral, scalp/hair and skin care products.

It is thus an object of the present invention to provide for a personal care composition where the actives included exhibit synergistic anti-inflammatory behavior.

It is another object of the present invention where at least one of the actives can be derived from a compound extractable from a natural source.

### Summary of the Invention

According to the first aspect of the present invention there is provided a personal care composition comprising:
(i) gingerdiol having the chemical structure; 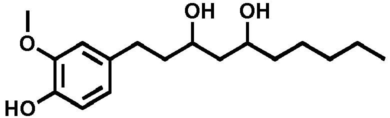 1-(4-hydroxy-3-methoxyphenyl)decane-3,5-diol
(ii) niacinamide; and
(iii) a cosmetically acceptable base.

According to another aspect of the present invention there is provided a process to prepare a composition of the first aspect comprising the steps of:
(i) Preparing gingerdiol by a process comprising the steps of (a) Taking gingerol in a suitable solvent and a reducing agent at a temperature of 0 to 10 °C (b) warming the reaction mixture to 15 to 25 °C to form the gingerdiol and (c) separating the gingerdiol from the mixture using solvent extraction; and
(ii) Mixing the product of step (i) with niacinamide and the cosmetically acceptable base.

According to yet another aspect of the present invention there is provided a method of reducing or preventing inflammation on a topical surface of a human or animal body comprising the steps of applying a composition of the first aspect on to the desired surface of the body.

### Detailed Description of the Invention

Except in the examples, or where otherwise explicitly indicated, all numbers in this description indicating amounts of material or conditions of reaction, physical properties of materials and/or use may optionally be understood as modified by the word "about". All amounts are by weight of the final composition, unless otherwise specified.

It should be noted that in specifying any ranges of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

"Oral care composition" for the purposes of the present invention means a paste, powder, liquid, gum, serum or other preparation for cleaning the teeth or other surfaces in the oral cavity.

"Tooth paste" for the purpose of the present invention means a paste or gel dentifrice for use with a toothbrush. Especially preferred are tooth pastes suitable for cleaning teeth by brushing for about two minutes.

"Mouth wash" for the purpose of the present invention means liquid dentifrice for use in rinsing the mouth. Especially preferred are mouth washes suitable for rinsing the mouth by swishing and/or gargling for about half a minute before expectorating.

Viscosity of a composition is the value taken at room temperature (25°C) with a Brookfield Viscometer, Spindle No.4 and at a speed of 5 rpm. Values are quoted in centipoises (cP=mPa.s) unless otherwise specified.

By a 'Hair Care Composition" as used herein, is meant to include a composition for topical application to hair and/or scalp of mammals, especially humans. Such a composition may be generally classified as leave-on or rinse off, and includes any product applied to a human body for also improving appearance, cleansing, odor control or general aesthetics. The composition of the present invention can be in the form of a liquid, lotion, cream, foam, scrub, gel, or bar. Non-limiting examples of such compositions include leave-on hair lotions, creams, and wash-off shampoos, conditioners, shower gels, or toilet bar. The composition of the present invention is preferably a wash-off composition, especially preferred being a shampoo or a conditioner and most preferably a shampoo.

"Water-insoluble", as used herein, refers to the solubility of a material in water at 25°C and atmospheric pressure being 0.1% by weight or less.

By 'A topical composition' or a 'skin care composition' as used herein, is meant to include a composition for topical application to the skin of mammals, especially humans. Such a composition could be of the leave-on or of the wash-off/ rinse-off type. By a leave-on composition is meant a composition that is applied to the desired skin surface and left on for a period of time (say from one minute to 24 hours) after which it may be wiped or rinsed off with water, usually during the regular course of personal washing. By a wash-off/ rinse off composition is meant a composition that is applied to the desired skin surface for a shorter period of time say of the order of seconds or minutes and usually contains sufficient surfactants that aids in cleaning the surface which may be rinsed off with copious amounts of water. The composition may also be formulated into a product which is applied to a human body for improving the appearance, cleansing, odor control or general aesthetics. The composition of the present invention can be in the form of a liquid, lotion, cream, foam, scrub, gel or stick form and may be delivered through a roll-on device or using a propellant containing aerosol can. "Skin" as used herein is meant to include skin on any part of the body e.g., neck, chest, back, arms, underarms, hands, legs, buttocks and scalp. When the product is used for the underarms it is usually called a deodourant procuct or a deo product. A class of deodourant product is the so called anti-perspirant (AP) product which contains an AP active which when applied to the axilla of an individual delivers anti-perspirancy and deodourancy benefits.

According to the first aspect of the invention there is provided a personal care composition comprising gingerdiol having the chemical structure; 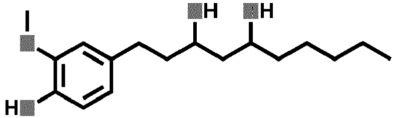 niacinamide and a cosmetically acceptable base.

Gingerdiol is also know by the chemical name 1-(4-hydroxy-3-methoxyphenyl)decane-3,5-diol. Gingerdiol is generally absent in extracts of natural material like ginger. If at all reported to be present, it is present in extremely trace amounts. Ginger is known by the botanical name *Zingiber officinale* and many different sub-species have been reported from many continents. Ginger has been used for a very long time as a food ingredient and is considered to be very safe due to its long history of use. Very many different ingredients isolated from extracts (using both aqueous and organic solvent extraction) of ginger have been known and used for very many cosmetic and pharmaceutical applications e.g. 4-gingerol, 6-gingerol, 8-gingerol, 1-gingerol, 6-shagaol, 8-shagaol, 10-shagaol etc. Gingerols and shagaol are known to be the major ingredients of actives from ginger which are biologically active. Ginger, in addition to use as a food ingredient had been used in traditional medicine to treat various disorders including cough, cold, nausea, and stomach pain.

6-gingerdiol is generally absent or present in less than 0.001% in ginger and therefore cannot be considered as naturally present in ginger. As per the present invention gingerdiol for use in the composition of the invention has to be synthesized in a chemical reaction. A suitable chemical synthesis process comprises the steps of taking gingerol in a suitable solvent and a reducing agent at a temperature of 0 to 10 °C; warming the reaction mixture to 15 to 25 °C to form the gingerdiol; and separating the gingerdiol from the mixture using solvent extraction.

The various possible chemicals and process conditions which may be used in the preparation of gingerdiol are given below:
Suitable solvents for the chemical synthesis include alcohols such as methanol, ethanol, isopropanol, n-butanol, sec-butanol, tert-butanol, amyl alcohol, isoamyl alcohol, tert-amyl alcohol, ethers such as tetrahydrofuran, 2-methyltetrahydrofuran, methyl tert-butyl ether, dioxane, diethoxymethane, monoglyme, diglyme, triglyme, tetraglyme, ethylene glycol monomethyl ether and miscellaneous solvents such as acetonitrile, dimethylformamide and dimethylsulfoxide and/or mixtures of these, preferably mixtures of alcohols and ethers, more preferably mixtures of methanol and tetrahydrofuran. Solvents do not necessarily have to be completely anhydrous and may contain small amounts of water. The concentration of gingerol in the solvent can range from very dilute (e.g. 0.01 M) to highly concentrated (e.g. 5 M), preferably in the range between 0.1 M to 2 M and more preferably between 0.5 M to 1 M. The initial temperature of the gingerol solution can range between 0 °C to ambient temperature (e.g. 21 °C), preferably between 5 °C to 15 °C. Cooling of the solution can be achieved using an ice bath or a low temperature recirculator bath. Stirring of the reaction can be achieved using a stir bar or a mechanical stirrer. The gingerol solution is preferably initially kept under an inert atmosphere using an inert gas such as nitrogen or argon and the inert atmosphere is then maintained throughout the entire reaction life cycle. Once the initial temperature and atmospheric conditions of the gingerol solution are achieved, an alkali metal borohydride reducing agent such as sodium borohydride, lithium borohydride, potassium borohydride, or an alkaline earth metal borohydride such as calcium borohydride or magnesium borohydride, preferably sodium borohydride (1.1 molar equivalent) is added in portions to maintain the reaction temperature below 30 °C throughout the reaction. After complete addition of the borohydride reagent, the reaction is allowed to warm up to ambient temperature over a period of up to 2 hour, preferably up to 1 hour. The progress of the reaction is monitored using thin layer chromatography for the formation of gingerdiol and complete disappearance of gingerol. Once the reaction is complete, the contents of the reaction are poured into ice cold saturated ammonium chloride (2 volumes) and the mixture extracted with an organic solvent (3 X 1 volume) such as ethyl acetate, diethyl ether, methyl tert-butyl ether, chloroform, dichloromethane, preferably ethyl acetate. The combined organic layers are dried using a drying agent such as sodium sulfate or magnesium sulfate, preferably sodium sulfate. Once the solution is dry, the drying agent is filtered off and the solvent removed under reduced pressure at < 45 °C to give crude gingerdiol. Crude gingerdiol is purified by chromatography on silica gel eluting with ethyl acetate:hexanes (1:1) to afford pure gingerdiol.

The appearance of pure gingerdiol prepared by this process is a thick pale yellow gel which is soluble in commonly used organic solvents such as alcohols, ethers, esters, acetonitrile, chloroform, dichloromethane, dimethylformamide, dimethylsulfoxide among others.

Once the gingerdiol is prepared, it is generally included at 0.01 to 5%, preferably at 0.1 to 3%, more preferably at 0.1 to 2% by weight of the personal care composition.

The personal care composition of the invention comprises niacinamide. Niacinamide has the structure given below:

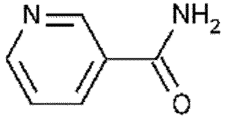

Niacinamide is preferably present in 0.01 to 5%, more preferably 0.1 to 3% by weight of the composition. The inventors have found that the combination of gingerdiol and niacinamide is able to provide synergistic anti-inflammatory benefit in a HGF (Human Gingival Fibroblast) invitro model.

When the personal care composition is delivered for oral care, it includes a cosmetically acceptable base which may be an abrasive, a thickener, a humectant or an orally acceptable surfactant.

### Oral Care

Oral care compositions preferably comprise an abrasive. Gels usually contain silica, whereas opaque creams generally contain calcium based abrasives, especially chalk.

Preferred toothpaste compositions have 5 to 60 wt% calcium based abrasive. In more preferred compositions it is 30 to 60 wt% and further more preferably from 35 to 55 wt%. Optimal compositions have 40 to 55wt% calcium based abrasive.

A preferred abrasive is fine ground natural chalk (FGNC), which is a form of chalk. It is obtained from limestone or marble. FGNC may also be modified chemically or physically by coating during milling or after milling by heat treatment. Typical coating materials include magnesium stearate or oleate. The morphology of FGNC may also be modified during the milling process by using different milling techniques, for example, ball milling, air-classifier milling or spiral jet milling.

FGNC may be used as the sole calcium based abrasive. However, FGNC may also be used with the other calcium based abrasives for some balance of abrasion. Usually the particle size of chalk is from 1 to 60 µm, and preferred sizes range from 1 to 15 µm.

Other preferred calcium based abrasives include dicalcium phosphate (DCP), calcium pyrophosphate and precipitated calcium carbonate (PCC), which preferably are included at 25 to 55 wt%, more preferably 35 to 50 wt%.

When a combination of calcium based abrasives is desired, it is preferred that FGNC is 35 to 100%, more preferably 75 to 100% and especially from 95 to 100% of the total amount of Calcium based abrasives. In such cases, the balance, most preferably, is PCC.

Other abrasives may also be used depending upon the intended degree of abrasion. These include synthetic abrasive polishing agents such as amorphous precipitated silica and silica gels. Other abrasive agents include magnesium carbonate, sodium metaphosphate, potassium metaphosphate, zirconium silicate, potassium metaphosphate, magnesium orthophosphate, tricalcium phosphate, magnesium orthophosphate, trimagnesium phosphate, aluminum silicate, zirconium silicate and perlite.

In an preferred embodiment, the composition comprises a thickener. Thickeners that may be used in this invention include, sodium carboxymethyl cellulose (SCMC), hydroxyl ethyl cellulose, methyl cellulose, ethyl cellulose, gum tragacanth, gum arabic, gum karaya, xanthan gum, sodium alginate, carrageenan gum, guar gum, Irish moss, starch, modified starch, silica based thickeners including silica aerogels, magnesium aluminum silicate (e.g., Veegum), Carbomers (cross-linked acrylates) and mixtures thereof.

Typically, thickening silica, sodium carboxymethyl cellulose and/or a Carbomer is/are preferred thickeners for use in the composition of the invention. When a Carbomer is employed, those having a weight-average molecular weight of at least 700,000 are desired, and preferably, those having a molecular weight of at least 1,200,000, and most preferably, those having a molecular weight of at least about 2,500,000 are desired. Mixtures of Carbomers may also be used herein.

In an especially preferred embodiment, the Carbomer is Synthalen PNC, Synthalen KP or a mixture thereof. It has been described as a high molecular weight and cross-linked polyacrylic acid and identified via CAS number 9063-87-0. These types of materials are available commercially from suppliers like Sigma.

In another especially preferred embodiment, the sodium carboxymethyl cellulose (SCMC) used is SCMC 9H. It has been described as a sodium salt of a cellulose derivative with carboxymethyl groups bound to hydroxy groups of glucopyranose backbone monomers and identified via CAS number 9004-32-4. The same is available from suppliers like Alfa Chem.

Thickening silica is especially preferred to be used in gel toothpastes. Gel toothpastes generally contain upto 8.5 wt% thickening silica whereas opaque toothpastes typically contain 3 to 4 wt% thickening silica.

When present, preferred thickening silicas include AEROSIL T series from Degussa or the CAB-O-SIL series from Cabot Corporation, silica gels such as the SYLODENT or SYLOX series from W. R.Grace & Co or precipitated silica such as ZEOTHIX 265 from J. M. Huber Corporation. Useful silica thickeners also include ZEODENT 165, ZEODENT 163 and/or 167 and ZEOFREE 153, 177, and/or 265 silicas, all available from J. M. Huber Corporation. Other preferred thickening silicas include MFIL, MFIL-P (From Madhu Silica, India), SIDENT 22 S and AEROSIL 200 (Ex. Evonik Industries), SYLODENTand PERKASIL thickening silicas from WR Grace & Company and Tixosil 43 and 331 from Rhodia, synthetic finely divided pyrogenic silica such as those sold under the trademarks SYLOID 244, SYLOID 266 and AEROSIL D- 200.

Thickener, when present, preferably makes up from 0.01 to about 10%, more preferably from 0.1 to 9%, and most preferably, from 1.5 to 8% by weight of the composition.

Suitable humectants are preferably used in the oral care composition of the present invention and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. Glycerin, polyethylene glycol, sorbitol or mixtures thereof are the preferred humectants.

The humectant may be present in the range of from 10 to 90% by weight of oral care compositions. More preferably, the humectant makes up from 25 to 80%, and most preferably, from 45 to 70% by weight of the composition, based on total weight of the composition and including all ranges subsumed therein.

Preferably, an oral care composition comprises a surfactant. Preferably the composition comprises at least 0.01% surfactant by weight of the composition, more preferably at least 0.1% and most preferably from 0.5 to 7%. Suitable surfactants include anionic surfactants, such as the sodium, magnesium, ammonium or ethanolamine salts of C8 to C18 alkyl sulphates (for example sodium lauryl sulphate), C8 to C18 alkyl sulphosuccinates (for example dioctyl sodium sulphosuccinate), C8 to C18 alkyl sulphoacetates (such as sodium lauryl sulphoacetate), C8 to C18 alkyl sarcosinates (such as sodium lauryl sarcosinate), C8 to C18 alkyl phosphates (which can optionally comprise up to 10 ethylene oxide and/or propylene oxide units) and sulphated monoglycerides. More preferably the surfactant comprises or is an anionic surfactant. The preferred anionic surfactants are sodium lauryl sulphate and/or sodium dodecylbenzene sulfonate. Most preferably the surfactant is sodium lauryl sulphate. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used. Most preferred surfactants are an alkali metal alkyl sulphate or a betaine.

Water may preferably be included in 5 to 95%, in particular 10 to 75%, and especially at from 10 to 60%, further more preferably 10 to 45% by total weight of the composition.

When the oral care composition of this invention is a toothpaste or gel, the same typically has a viscosity from about 30,000 to 180,000 centipoise, and preferably, from 60,000 to 170,000 centipoise, and most preferably, from 65,000 to 165,000 centipoise.

The oral care composition of the present invention may contain a variety of other ingredients which are common in the art to enhance physical properties and performance. These ingredients include antimicrobial, anti-caries agents, plaque buffers, fluoride sources, vitamins, plant extracts, desensitizing agents, anti-calculus agents, biomolecules, flavors, proteinaceous materials, preservatives, opacifying agents, coloring agents, pH-adjusting agents, sweetening agents, particulate abrasive materials, polymeric compounds, buffers and salts to buffer the pH and ionic strength of the compositions, and mixtures thereof. Such ingredients typically and collectively make-up less than 20% by weight of the composition, and preferably, from 0.0 to 15% by weight, and most preferably, from 0.01 to 12% by weight of the composition, including all ranges subsumed therein.

### Hair Care

As per a preferred aspect of the invention, the composition may be used for hair care. It is especially useful for preventing or alleviating the symptoms of dandruff. One medium through which this may be delivered is that of a shampoo. The composition of the invention especially shampoos are formulated with an anionic surfactant e.g. an alkyl sulphate and/or ethoxylated alkyl sulfate surfactant. These anionic surfactants are preferably present at a level of from 1 to 20%, preferably 2 to 16%, further more preferably from 3 to 16% by weight of the composition. Preferred alkyl sulfates are C8-18 alky sulfates, more preferably C12-18 alkyl sulfates, preferably in the form of a salt with a solubilising cation such as sodium, potassium, ammonium or substituted ammonium.

Preferred alkyl ether sulfates are those having the formula: RO(CH₂CH₂O)ₙSO₃M; wherein R is an alkyl or alkenyl having from 8 to 18 (preferably 12 to 18) carbon atoms; n is a number having an average value of greater than at least 0.5, preferably between 1 and 3, more preferably between 2 and 3; and M is a solubilising cation such as sodium, potassium, ammonium or substituted ammonium. An example is sodium lauryl ether sulfate (SLES).

Preferred ethoxylated alkyl sulfate anionic surfactant is sodium lauryl ether sulfate (SLES). SLES having an average degree of ethoxylation of from 0.5 to 3, preferably 1 to 3 is especially preferred.

Shampoo compositions according to the invention may comprise one or more further anionic cleansing surfactants which are cosmetically acceptable and suitable for topical application to the hair.

A composition of the invention preferably additionally comprises an amphoteric surfactant preferably a betaine surfactant preferably an alkyl amidopropyl betaine surfactant for example cocamidopropyl betaine. In a preferred embodiment, the composition comprises from 0.1 to 10 wt.%, preferably from 0.5 to 8 wt.%, more preferably from 1 to 5 wt.% of a betaine surfactant

To enhance deposition of actives from compositions of the invention especially shampoos, cationic polymers are generally included therein. In the present invention too, it is preferred that the composition additionally includes 0.01 to 2.0% of a cationic polymer. The cationic polymer is preferably guar hydroxypropyl trimonium chloride. Guar polymer predominantly contains galactomannan polymer chains. This polymer is available at various molecular weights and degree of cationic substitutions depending on how much the guar has been hydrolysed and cationised. The cationic polymer is preferably present in 0.04 to 0.5%, more preferably 0.08 to 0.25% by weight of the composition.

When conditioning benefits are to be delivered through the composition of the invention the composition is called a hair conditioner. Typically, the most popular conditioning agents used in hair care compositions are water-insoluble oily materials such as mineral oils, naturally occurring oils such as triglycerides and silicone polymers. Conditioning benefit is achieved by the oily material being deposited onto the hair resulting in the formation of a film, which makes the hair easier to comb when wet and more manageable when dry. An especially useful conditioning agent is a silicone compound, preferably a non-volatile silicone compound. Advantageously compositions herein may include one or more silicones. The silicones are conditioning agents found in dispersed or suspended particulate form. They are intended to deposit onto hair remaining behind after rinsing of the hair with water. Suitable silicone oils may include polyalkyl siloxanes, polyaryl siloxanes, polyalkylaryl siloxanes, polyether siloxane copolymers and mixtures thereof. Amino silicones are often formulated with shampoo compositions. Amino silicones are silicones containing at least one primary amine, secondary amine, tertiary amine or a quaternary ammonium group. High molecular weight silicone gums can also be utilized. Another useful type are the crosslinked silicone elastomers such as Dimethicone/Vinyl/Dimethicone Crosspolymers (e.g. Dow Corning 9040 and 9041).

Amounts of the silicone in compositions where present may range from about 0.1 to about 10 wt.%, preferably from about 0.1 to about 8wt.%, more preferably from about 0.3 to about 5wt.% by weight of the hair care compositions.

The pH of the composition is preferably equal to or higher than 4.0, more preferably in the range of 5.0 to 7.0.

The hair conditioning composition usually comprises conditioning surfactants selected from cationic surfactants, used singly or in admixture. Suitable cationic surfactants for use in conditioner compositions according to the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, dihydrogenated tallow dimethyl ammonium chloride (eg, Arquad 2HT/75 from Akzo Nobel), cocotrimethylammonium chloride, PEG-2-oleammonium chloride and the corresponding hydroxides thereof. Further suitable cationic surfactants include those materials having the CTFA designations Quaternium-5, Quaternium-31 and Quaternium-18. Mixtures of any of the foregoing materials may also be suitable. A particularly useful cationic surfactant for use in conditioners according to the invention is cetyltrimethylammonium chloride, available commercially, for example as GENAMIN CTAC, ex Hoechst Celanese. Another particularly useful cationic surfactant for use in conditioners according to the invention is behenyltrimethylammonium chloride, available commercially, for example as GENAMIN KDMP, ex Clariant. Yet another preferred cationic surfactant is stearamidopropyl dimethylamine.

The most preferred cationic surfactants for use in the composition are stearamidopropyl dimethylamine, behentrimonium chloride, or stearyl trimethyl ammonium chloride. In conditioners of the invention, the level of cationic surfactant will generally range from 0.1 % to 5%, preferably 0.5 to 2.5% by weight of the composition.

Hair conditioning compositions of the invention preferably may also additionally comprise a fatty alcohol. The combined use of fatty alcohols and cationic surfactants in conditioning compositions is believed to be especially advantageous, because this leads to the formation of a lamellar phase, in which the cationic surfactant is dispersed.

Representative fatty alcohols comprise from 8 to 22 carbon atoms, more preferably 16 to 22. Fatty alcohols are typically compounds containing straight chain alkyl groups. Examples of suitable fatty alcohols include cetyl alcohol, stearyl alcohol and mixtures thereof. The use of these materials is also advantageous in that they contribute to the overall conditioning properties of compositions of the invention.

The level of fatty alcohol in conditioners of the invention will generally range from 0.5 to 10%, preferably from 0.1 % to 8%, more preferably from 0.2 % to 7 %, most preferably from 0.3 % to 6 % by weight of the composition. The weight ratio of cationic surfactant to fatty alcohol is suitably from 1:1 to 1:10, more preferably from 1:1.5 to 1:8, optimally from 1:2 to 1:5.

Hair care compositions whether delivered as shampoos or conditioners usually comprise an anti-dandruff agent. The most preferred anti-dandruff agent for use in the compositon of the invention is a zinc based anti-dandruff agent preferably zinc pyrthione. Zinc pyrithione belongs to the class of insoluble metal pyrithione which may be represented by the following general formula: 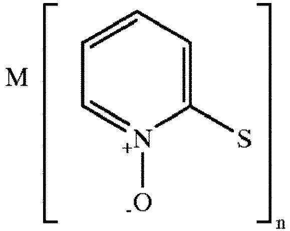 in which M is a polyvalent metal ion and n corresponds to the valency of M. In the present invention M corresponds to Zinc and n has the value of 2.

The zinc pyrithione may have any particle form suitable for use in a composition for topical application. For example, the zinc pyrithione may be in the form of amorphous or crystalline particles having a range of different particle sizes. The zinc pyrithione may, for example, be in the form of particles having a size distribution in which at least about 90% of the particles have a size of up to 100 microns, more preferably up to 50 microns, even more preferably up to 10 microns, most preferably 5 microns or less.

Various methods for producing fine particles of metal pyrithione are described, for example, in EP-A-0 173 259. Suitable methods for determining particle size are described in that document. The insoluble metal pyrithione may be made up of one particulate form or two or more different particulate forms.

Other suitable particulate forms for the zinc pyrithione include platelets and needle-shaped particles. Platelets of zinc pyrithione are described in EP-A-0034385, the contents of which are incorporated herein by reference. The needleshaped particles are preferably of the type described in WO99/66886, the contents of which are incorporated herein by reference. For needle-shaped particles preferably at least 50% by number of the particles are needle-shaped particles having a length of between 1 µm and 50 µm.

The amount of metal pyrithione incorporated into the compositions may depend on the type of composition and the exact nature of the material used. A preferred amount of pyrithione is from about 0.01% to about 1.5% by weight of the total composition, more preferably from about 0.05% to about 1.5% by weight of the total composition.

The composition as per the invention especially for anti-dandruff shampoos preferably additionally comprises a zinc compound. The presence of additional zinc compound in the composition is believed to improve the antidandruff efficacy of the zinc based antidandruff agent. Suitable zinc compounds are zinc oxide, zinc citrate, zinc malonate, zinc carbonate or combinations thereof. The zinc compound is preferably present in 0.1 to 3%, more preferably 0.1 to 1.5% by weight of the composition.

Shampoo composition as per the invention preferably additionally comprises a conazole fungicide. Preferably the conazole fungicide is selected form ketoconazole, climbazole or mixtures thereof. The azole fungicide is preferably included in 0.01 to 2%, more preferably 0.025 to 0.75% by weight of the composition. The presence of a conazole fungicide is believed to improve the deposition of zinc pyrithione.

### Skin Care

The composition of the invention may be used for skin care. The cosmetically acceptable base in such cases may be a liquid or solid material. Typically, base is present in an amount ranging from 10 to 99.9%, more preferably from 20 to 95%, most preferably from 40 to 85% by total weight of the composition including all ranges subsumed therein. It is particularly preferred that the cosmetically acceptable carrier includes water. Water is preferably included in an amount from 30 to 90%, more preferably from 30 to 85%, most preferably from 30 to 80% by total weight of the sunscreen composition. Besides water, suitable carrier classes include silicones, polyhydric alcohols, hydrocarbons, triglycerides and thickening powders.

The skin care composition of the invention may be in any form including toners, lotions, creams, mousses, scrub, serum or gel that is suitable for topical application to the skin. The composition can be either a leave-on product such as skin lotions, creams, antiperspirants, deodorants, lipsticks, foundations, mascara, sunless tanners and sunscreen lotions or a rinse-off product such as shower gels and toilet bars. It is preferred that the composition is a skin lotion or a cream.

The composition may comprise an emollient oil that act as a co-solvent. Suitable emollient oils include, for example, ester of alkoxylated aromatic alcohol with fatty carboxylic acid, esters of polyglycols or diols with fatty carboxylic acid such as caprylic/capric triglyceride, ester of fatty alcohol and fatty acid, alkoxylated derivative of benzyl alcohol and mixtures thereof. Preferably the emollient oil is caprylic/capric triglyceride.

Typically, such compositions comprise co-solvent in an amount from 0.01 to 10%, more preferably from 0.1 to 8%, most preferably from 1 to 6%, based on the total weight of the sunscreen composition and including all ranges subsumed therein.

The composition may additionally comprise sunscreen agents such as inorganic sunscreens. For example, zinc oxide, titanium dioxide, iron oxide, silica such as fumed silica. The amount of such sunscreen agents is preferably incorporated from 0.1 to 5% by total weight of the sunscreen composition.

The composition of the invention may comprise a UV-A sunscreen agent selected from the group consisting of a dibenzoylmethane derivative, a triazine derivative, a benzophenone derivative and mixtures thereof. In a preferred embodiment, the UV-A sunscreen agent comprises or is a dibenzoylmethane derivative, for example, butyl methoxydibenzoylmethane (sold under the trade name Parsol 1789).

Typically, the sunscreen composition of the present invention comprises from 0.1 to 15% by weight of the UV-A sunscreen agent, more preferably from 0.1 to 10%, most preferably from 1 to 5%, based on the total weight of the composition and including all ranges subsumed therein.

The composition of the invention may also comprise a UV-B sunscreen agent. Suitable UV-B sunscreen agent of the invention is selected from the group consisting of a benzophenone, an anthranilate, a salicylate, a cinnamate, a camphor, benzylidene malonate, a triazone, and derivatives thereof. In a preferred embodiment, the UV-B sunscreen agent comprises or is a cinnamate derivative, for example, ethylhexyl methoxycinnamate (sold under the trade name Parsol MCX).

Typically, the composition comprises from 0.1 to 20% by weight of the UV-B sunscreen agent, more preferably from 0.5 to 18%, most preferably from 1 to 15%, based on the total weight of the composition and including all ranges subsumed therein.

A skin lightening agent may also be incorporated into the composition of the invention. Suitable skin lightening agents other than Vitamin B3 and its derivatives (e.g. niacin, nicotinic acid, niacinamide) are kojic acid, arbutin, tranexamic acid, placental extract, ascorbic acid and its derivatives (e.g. magnesium ascorbyl phosphate, sodium ascorbyl phosphate, ascorbyl glucoside, and ascorbyl tetraisopalmitates), aloe extract, ammonium lactate, azelaic acid, citrate esters, ellagic acid, glycolic acid, green tea extract, hydroquinone, lemon extract, linoleic acid, vitamins like vitamin B6, vitamin B12, vitamin C, vitamin A, a dicarboxylic acid, resorcinol derivatives, hydroxycarboxylic acid like lactic acid and their salts (e.g. sodium lactate) or a mixture thereof. Typically the skin lightening agent is present in an amount from 0.1 to 10%, more preferably from 0.2 to 5%, most preferably from 0.3 to 3% by total weight of the composition including all ranges subsumed therein.

The composition may also comprise other ingredients which are common in the art to enhance physical properties and performance. Suitable ingredients include but are not limited to humectants, thickeners, opacifiers, binders, colorants and pigments, pH adjusting agents, preservatives, optics, perfumes, viscosity modifiers, biological additives, buffering agents, conditioners, natural extracts, essential oils and skin benefit agents including anti-inflammatory agents, cooling agents, antiperspirant agents, anti-aging agents, anti-acne agents, anti-microbial agents and antioxidants.

The invention will now be illustrated with the help of the following non-limiting example.

### Examples

### Examples 1-4: Synergistic anti-inflammation efficacy of 6-gingerdiol with niacinamide in THP-1 invitro assay

The following procedure was used to test the anti-inflammation efficacy of the various actives alone or in combination.

THP1-XBlue™ (Cat No: thpx-sp, InvivoGen) cells were cultured as suspense in RPMI 1640 medium supplemented with 10% FBS, penicillin (10 U/mL) - streptomycin (10 µg/ML). Cells were differentiated in 24-well plates at the density of 5 x 10⁵ cells / well with 100 nM PMA for 72 h. Cells were then co-treated with pure *E*. *coli* lipopolysaccharides (LPS) and single concentration or a combination of 6-gingerdiol and niacinamide at different doses. After 24 h, the supernatants were collected measured for interleukin (IL)-6 as pro-inflammatory bio-marker using enzyme-linked immunosorbent assay (ELISA).

The results in terms of concentration of IL-6 in pg/ml is given in Table -1 below:

**Table 1**

| **Examples** | **Composition** | **Concentration of IL-6 (pg/ml)** | **Std. dev** |
|---|---|---|---|
| 1 | LPS | 10547 | 311 |
| 2 | 10 µM of 6-gingerdiol | 8781 | 522 |
| 3 | 16 mM of niacinamide | 6031 | 318 |
| 4 | 10 µM of 6-gingerdiol + 16 mM of niacinamide | 4716 | 382 |

### Examples 5-10: Synergistic anti-inflammation efficacy of 6-gingerdiol with niacinamide in THP-1 invitro assay at various other concentrations

Experiments similar to that in Table - 1 were carried out and similar synergistic data was obtained over various other concentrations. The data is summarised in Table - 2 below:

**Table 2**

| Examples | Composition | Concentration of IL-6 (pg/ml) | Std. dev |
|---|---|---|---|
| 5 | LPS | 46137 | 4279 |
| 6 | 1.25 µM of 6-gingerdiol | 37212 | 4960 |
| 7 | 1 mM of niacinamide | 39894 | 4587 |
| 8 | 0.5 mM niacinamide | 41688 | 9285 |
| 9 | 1.25 µM of 6-gingerdiol + 1 mM of niacinamide | 32370 | 3068 |
| 10 | 1.25 µM of 6-gingerdiol + 0.5 mM of niacinamide | 28988 | 723 |

The data in Table 1 and 2 indicate the synergistic efficacy of a combination of niacinamide with 6-gingerdiol and this is applicable over a wide range of concentrations.

### Examples 11-20: Synergistic anti-inflammation efficacy of 6-gingerdiol with niacinamide in 2-D Dandruff Model at various other concentrations:

Primary adult human keratinocytes (HEKa) frozen at passage 1 were purchased from Invitrogen (Cat no C0055C). They were thawed into KGM Gold (Lonza cat no 00195769) made up with all components of the accompanying bullet kit plus 70µM calcium (from a stock solution of 200mM calcium chloride), placed in a T75 tissue culture flask and grown to 70-80% confluence. The cells were passaged and again grown to 70-80% confluence. At passage 3 the cells were washed with calcium and magnesium free PBS (Life Technologies cat no 14190-094) and removed from the flask using trypsin-EDTA (Life Technologies cat no 25300-054) and washed by centrifugation with trypsin neutralising solution (Life Technologies cat no R-002-100). The cell pellet was re-suspended in KGM Gold plus 70µM calcium and containing the bullet kit components but omitting the gentamycin and hydrocortisone (referred to as plating medium). The cells were plated into 24-well plates at a density of 2x10⁴ cells/well and allowed to settle for 24 hours prior to treatment.

The medium is removed from the cells and replace by 0.5ml plating medium containing 200µg/ml IL-17, 200µg/ml IL-22 and 10µg/ml TNFα together with the active at the required concentration. A concentration range can be done. Also included are an untreated control, a vehicle control and a triple cytokine mix without active all in triplicate. The plates are incubated for a further 16 to 24 hours. In this case the actives were 6-gingerdiol and a selection of curcuminoids.

The medium is harvested for IL-8 ELISA (R &D Systems Duoset kit cat no DY208) and the cells lysed for protein assay (Pierce BCA Protein Assay Kit Life Technologies cat no 23225) in order to normalise IL-8 to protein.

The results in terms of concentration of IL-8 in pg/100µgprotein is given in Table -3 below

**Table 3**

| Examples | Composition | Concentration of IL-8 (pg/100µgprotein) | Std. dev |
|---|---|---|---|
| 11 | control | 16.45 | 4.97 |
| 12 | vehicle control | 11.96 | 1.43 |
| 13 | triple cytokine mix | 744.83 | 170.58 |
| 14 | 10 mM of niacinamide | 408.30 | 76.80 |
| 15 | 5 µM of 6-gingerdiol | 347.99 | 96.59 |
| 16 | 5 µM of 6-gingerdiol +10 mM of niacinamide | 304.01 | 40.46 |
| 17 | 10 µM of 6-gingerdiol | 446.85 | 126.18 |
| 18 | 10 µM of 6-gingerdiol +10 mM of niacinamide | 186.50 | 5.46 |
| 19 | 20 µM of 6-gingerdiol | 390.31 | 243.43 |
| 20 | 20 µM of 6-gingerdiol +10 mM of niacinamide | 323.70 | 27.82 |

The data in Table 3 indicates the synergistic efficacy of a combination of niacinamide with 6-gingerdiol and this is applicable over a wide range of concentrations.

### Example 21: Synthesis of gingerdiol: Gingerdiol for use in Table 1 and 2 above were synthesized using the following procedure:

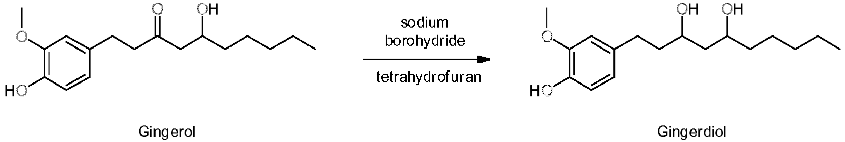

Gingerol (17.3g, 58.7mmol) was dissolved in anhydrous tetrahydrofuran (450ml) and cooled to 5 °C. Sodium borohydride (2.4g, 63mmol) was carefully added in portions, keeping the temp at 5 °C. The solution was allowed to warm up to 19 °C over 1h. At this time, thin layer chromatography on silica gel eluting with ethyl acetate:hexanes (1:1) showed the clean formation of gingerdiol and no gingerol remaining. The reaction mixture was poured into ice cold saturated ammonium chloride (500ml) and extracted with ethyl acetate (500ml). The layers were separated and the aqueous layer extracted once more with ethyl acetate (500ml). The combined organic layers were dried using sodium sulfate, filtered and the solvents removed in vacuo to give a yellow oil (20.1g). The crude product was purified by flash chromatography on silica gel eluting with ethyl acetate:hexanes (1:1) to afford pure product as a pale-yellow thick gel (16.7g, 96% yield). HPLC-UV (281nm) showed 98.7% purity. LC-MS (ESI⁺) showed expected mass [M+H]⁺ 295.2 (100%); ¹H NMR (60 MHz, CDCl₃) δ 6.68-6.74 (3H, m, -C=CH), 5.54 (1H, s, Ar-OH), 3.82-4.07 (5H, m, Ar-OCH₃, CH-OH), 2.51-2.77 (2H, m, Ar-CH₂-), 1.30-1.90 (12H, m, -CH₂-), 0.89-1.04 (3H, m, -CH₃); ¹³C NMR (60 MHz, CDCl₃) δ 149.4, 146.6, 136.8, 123.7, 117.3, 114.1, 75.9, 72.1, 58.6, 42.1, 41.7, 39.6, 34.5, 33.8, 28.2, 25.4, 16.9.

## Claims

1. A personal care composition comprising:
(i) gingerdiol having the chemical structure; 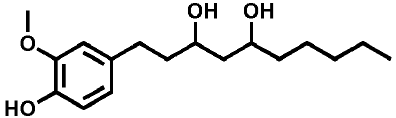 1-(4-hydroxy-3-methoxyphenyl) decane-3,5-diol;
(ii) niacinamide; and
(iii) a cosmetically acceptable base.

2. A composition as claimed in claim 1 comprising 0.01 to 5% gingerdiol.

3. A composition as claimed in any one of the preceding claims comprising 0.01 to 5% niacinamide by weight of the composition.

4. A composition as claimed in any one of the preceding claims in the form of an oral care, or a skin, scalp or hair care product.

5. A process to prepare a composition as claimed in claim 1 comprising the steps of:
(i) preparing gingerdiol by a process comprising the steps of (a) taking gingerol in a suitable solvent and a reducing agent at a temperature of 0 to 10°C (b) warming the reaction mixture to 15 to 25°C to form the gingerdiol and (c) separating the gingerdiol from the mixture using solvent extraction; and
(ii) mixing the product of step (i) with the niacinamide and the cosmetically acceptable base.

6. A process as claimed in claim 5 wherein the solvent is an alcohol, an ether or a mixture thereof; preferably a mixture of tetrahydrofuran and methanol.

7. A process as claimed in claim 5 or 6 wherein the reducing agent is sodium borohydride.

8. A personal care composition as claimed in any one of the preceding claims 1 to 4 for use as a medicament.

9. A personal care composition as claimed in any one of the preceding claims 1 to 4 comprising:
(i) gingerdiol having the chemical structure; 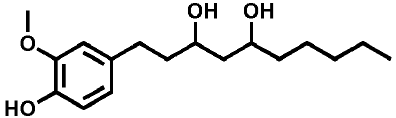 1-(4-hydroxy-3-methoxyphenyl) decane-3,5-diol;
(ii) niacinamide; and
(iii) a cosmetically acceptable base,
for use in reducing or preventing inflammation on a topical surface of a human or animal body comprising the step of applying the composition to the desired surface.

## Patentansprüche

1. Körperpflegezusammensetzung, umfassend:
(i) Gingerdiol mit der chemischen Struktur; 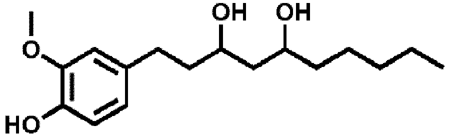 1-(4-Hydroxy-3-methoxyphenyl)decan-3,5-diol;
(ii) Niacinamid; und
(iii) eine kosmetisch akzeptable Basis.

2. Zusammensetzung wie in Anspruch 1 beansprucht, umfassend 0,01 bis 5% Gingerdiol.

3. Zusammensetzung wie in irgendeinem der vorhergehenden Ansprüche beansprucht, umfassend 0,01 bis 5% Niacinamid, bezogen auf das Gewicht der Zusammensetzung.

4. Zusammensetzung wie in irgendeinem der vorhergehenden Ansprüche beansprucht, in Form einer Mundpflege oder eines Haut-, Kopfhaut- oder Haarpflegeprodukts.

5. Verfahren zur Herstellung einer Zusammensetzung wie in Anspruch 1 beansprucht, umfassend die Schritte:
(i) Herstellen von Gingerdiol nach einem Verfahren, umfassend die Schritte (a) des Aufnehmens von Gingerol in ein geeignetes Lösungsmittel und ein Reduktionsmittel bei einer Temperatur von 0 bis 10 °C (b) des Erwärmens des Reaktionsgemisches auf 15 bis 25 °C zur Bildung von Gingerdiol und (c) des Trennens des Gingerdiols von der Mischung unter Verwendung von Lösungsmittelextraktion; und
(ii) Mischen des Produkts von Schritt (i) mit dem Niacinamid und der kosmetisch akzeptablen Basis.

6. Verfahren wie in Anspruch 5 beansprucht, wobei das Lösungsmittel ein Alkohol, ein Ether oder eine Mischung davon ist; bevorzugt eine Mischung aus Tetrahydrofuran und Methanol.

7. Verfahren wie in Anspruch 5 oder 6 beansprucht, wobei das Reduktionsmittel Natriumborhydrid ist.

8. Körperpflegezusammensetzung wie in irgendeinem der vorhergehenden Ansprüche 1 bis 4 beansprucht, zur Verwendung als Arzneimittel.

9. Körperpflegezusammensetzung wie in irgendeinem der vorhergehenden Ansprüche 1 bis 4 beansprucht, umfassend:
(i) Gingerdiol mit der chemischen Struktur; 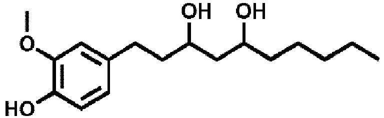 1-(4-Hydroxy-3-methoxyphenyl)decan-3,5-diol;
(ii) Niacinamid; und
(iii) eine kosmetisch akzeptable Basis,
zur Verwendung beim Reduzieren oder Verhindern einer Entzündung auf einer topischen Oberfläche eines menschlichen oder tierischen Körpers, umfassend den Schritt des Aufbringens der Zusammensetzung auf die gewünschte Oberfläche.

## Revendications

1. Composition pour le soin de la personne comprenant :
(i) un gingerdiol ayant la structure chimique ; 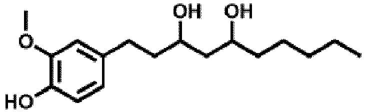 1-(4-hydroxy-3-méthoxyphényl)décane-3,5-diol;
(ii) un niacinamide ; et
(iii) une base cosmétiquement acceptable.

2. Composition selon la revendication 1 comprenant de 0,01 à 5 % de gingerdiol.

3. Composition selon l'une quelconque des revendications précédentes comprenant de 0,01 à 5 % de niacinamide en masse de la composition.

4. Composition selon l'une quelconque des revendications précédentes dans la forme d'un soin oral, ou d'un produit pour le soin de la peau, du cuir chevelu ou des cheveux.

5. Procédé de préparation d'une composition selon la revendication 1 comprenant les étapes de :
(i) préparation de gingerdiol par un procédé comprenant les étapes de (a) prise de gingerdiol dans un solvant approprié et un agent réducteur à une température de 0 à 10°C (b) chauffage du mélange réactionnel à de 15 à 25°C pour former le gingerdiol et (c) séparation du gingerdiol à partir du mélange en utilisant une extraction au solvant ; et
(ii) mélange du produit de l'étape (i) avec le niacinamide et la base cosmétiquement acceptable.

6. Procédé selon la revendication 5, dans lequel le solvant est un alcool, un éther ou un mélange de ceux-ci ; de préférence un mélange de tétrahydrofurane et méthanol.

7. Procédé selon la revendication 5 ou 6, dans lequel l'agent réducteur est le borohydrure de sodium.

8. Composition pour le soin de la personne selon l'une quelconque des revendications 1 à 4 précédentes pour une utilisation comme un médicament.

9. Composition pour le soin de la personne selon l'une quelconque des revendications 1 à 4 précédentes comprenant :
(i) un gingerdiol ayant la structure chimique ; 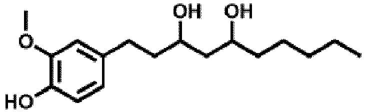 1-(4-hydroxy-3-méthoxyphényl)décane-3,5-diol;
(ii) un niacinamide ; et
(iii) une base cosmétiquement acceptable,
pour une utilisation dans la réduction ou la prévention d'inflammation sur une surface topique d'un corps humain ou animal comprenant l'étape d'application de la composition sur la surface souhaitée.
